# EUROPEAN PATENT APPLICATION

(11) **EP 3 085 244 A1**
(43) Date of publication of application: **26.10.2016**
(21) Application number: 15461539.7
(22) Date of filing: 17.06.2015
(51) Int. Cl.: A23L 3/00, A23L 3/32, A23B 7/015, A61L 2/03, A61L 2/14, B01J 19/08, H05H 1/24

(54) **NON-THERMAL PLASMA REACTOR FOR THE STERILIZATION OF ORGANIC PRODUCTS**

(30) Priority: 22.04.2015 PL 41203215
(71) Applicant: Zachodniopomorski Uniwersytet Technologiczny w Szczecinie, 70-310 Szczecin (PL)
(72) Inventor: Grabowski, Maciej, 71-687 Szczecin (PL); D browski, Waldemar, 71-215 Szczecin (PL); Balcerak, Micha, 70-543 Szczecin (PL); Ho ub, Marcin, 71-499 Szczecin (PL); Kalisiak, Stanis aw, 71-312 Szczecin (PL); Jakubowski, Tomasz, 71-374 Szczecin (PL); Chyla, Mariusz, 70-390 Szczecin (PL)
(74) Representative: Zawadzka, Renata

(57) **Abstract**

The non-thermal plasma reactor for organic product sterilization, especially for spices, according to the invention, equipped with a chamber, base (1), on which a cover (2) of the reactor is placed, a grounding bottom electrode (3), and an upper electrode (4), characterizes in that it has a mobile upper multi-needle electrode (4) connected via insulators (5) with a slide mechanism (6), which is placed on the base (1) on the bottom insulators (7). The lower electrode (3) has perforations. The reactor power is from 0 to 350 W and voltage of 0 up to 30 kV.

## Description

The present invention is a non-thermal plasma reactor for organic products sterilization, especially spices, induced in a gaseous medium (modified according to the sterilized product) in an inter-electrode space. The electrodes are supplied with a high voltage pulse of a variable shape, amplitude and a frequency repetition. Such variability of power supply parameters allows to modify the non-thermal plasma in a flexible manner.

The spices which are commercially available are not sterile. Microbiological analyses showed that the total number of non-spore forming aerobic bacteria is 10⁵ cfu/g, the total number of aerobic spore-forming bacteria is 10⁵ cfu/g, and the total number of anaerobic spore-forming bacteria is 10⁴ cfu/g. It is known to sterilize spices by means of saturated steam at pressure from 0.16 to 0.60 bar and a temperature from 120 to 400°C. The sterilization process is carried out from 15 to 60 seconds. The applied sieves additionally separate the impurities from the sterilized raw material. The use of magnets allows to remove the ferromagnetic materials from the raw material. The patent description PL189396 is known as a method of continuous sterilization of spices and other vegetable raw materials. The material moves in a continuous longitudinal-transversal movement in a gutter-shaped reactor equipped with a twin screw conveyor. During the movement of the raw material, it is subjected to the action of either saturated or superheated steam, which is released directly from the power nozzle placed inside the gutter-shaped reactor. The resulting intermediate product is dried to a final product with balanced moisture. The Polish patent application P388955 describes microwave apparatus for sterilizing herbs, spices, tobacco, dried vegetables and fruit, in which the sterilized material is heated by microwave energy with a frequency from 900 MHz to 3000 MHz in a metal chamber with a dielectric conveyor belt or a drum located inside. After adjusting the temperature to the range from 70°C to 130°C, the sterilized material is introduced into the cooling chamber. In this chamber, the material is cooled down to the temperature below 15°C in less than three minutes in a cold air stream. The cooling chamber can be made in the form of a vibrating conveyor with a forced flow of cold air, in the form of a sieve with a forced flow of cold air from the bottom of the sieve, or in the form of a pneumatic conveyor system in which the material is transported in a stream of cold air forced by a fan through an air cooling system. From the Polish patent application P408384, a method of sterilizing spices by means of cold plasma is known, wherein the sterilization is carried out under atmospheric pressure of either argon or air, at a temperature varied from 19 to 85 °C. From the Polish patent application P405861 is known that the spices are dried at temperature from 25°C to 35°C for a period from 2 hours to 3.5 hours or until they reach the water activity value below 0,300. Next, the dried spice is subjected to the low pressure cold plasma in the presence of gaseous hydrogen peroxide concentration from 50% to 70%. The hydrogen peroxide which enters the plasma is always in the form of gas.

In the existing non-thermal plasma reactors for organic products sterilization, such as generating corona discharge (Gurol C., Ekinci F., Aslan N., Korachi M. 2012. Low temperature plasma for decontamination of E. coli in milk. International Journal of Food Microbiology 157:1 - 5; Bermúdez-Aguirre D., Wemlinger E., Pedrow P., Barbosa-Cánovas G., Garcia-Perez M. 2013. Effect of atmospheric pressure cold plasma (APCP) on the inactivation of Escherichia coli in fresh produce. Food Control 34:149 - 157) and dielectric barrier discharge (Rød S., Hansen F., Leipold F., Knøchel S. 2012. Cold atmospheric pressure plasma treatment of ready-to-eat meat: Inactivation of Listeria inocua and changes in product quality. Food Microbiology 30:233 - 238; Choi J., Han I., Baik H, Lee M., Han D., Park J., Lee I., Song K., Lim Y. 2006. Analysis of sterilization effect by pulsed dielectric barrier discharge. Journal of Electrostatics 64:17 - 22; Dobrynin D., Friedman G., Fridman A., Starikovskiy A. 2011. Inactivation of bacteria using dc corona discharge role of ions and humidity. New Journal of Physics 13:1-13) the reactor constructions do not allow to carry out the process in an optimal way. The reason of this is the fact that during the discharge process a smooth change of the position of the electrode in relation to the product is not possible. The mutual change of the position of the electrodes and the batch has a significant impact on the quality and the effectiveness of the sterilization process. The description of the invention US20100015358 A1 reveals a system of cooling a plasma reactor by means of a coolant and a cooling radiator. In most cases, reactors are switched off for a while to cool down before the plasma sterilization process is continued.

The non-thermal plasma reactor for organic products sterilization, in particular for spices, according to the invention, is equipped with a chamber, a base, on which a housing is placed, a grounding lower and an upper electrode. It characterizes in that it has a mobile multi-needle upper electrode connected via insulators with its sliding mechanism which is placed on the base on the bottom insulators. The upper electrode is placed on an insulated mobile platform and is joined with electrical culvert with high potential. The bottom electrode has perforations (e.g. grid or plate), and is connected to the grounding potential. The reactor has power of 0 to 350 W and voltage of 0 to 30 kV. At least one Peltier module is placed on the housing of the reactor, above the upper electrode. This enables cooling of the reactor without the use of cooling liquid.
The reactor preferably has at least one fan, placed on the housing above the upper electrode, for cooling the Peltier module.
Generating plasma in the chamber is carried out by administering high-voltage on the electrode system. Preferably, a dielectric (made of e.g. stainless steel, glass, Al₂O₃) is placed below the upper electrode so that dielectric barrier discharge is generated instead of corona discharge. This allows the excitation of two types of discharges in one plasma reactor. In other words, in one reactor, corona discharge is generated by the multi-needle electrode, whereas by placing a flat plate (the dielectric mentioned above) under the needles, the dielectric barrier discharge will be received. In the case of dielectric barrier discharge, the electrode is flat, and therefore the alternative method of plasma excitation is used. The applied dielectric moves up and down together with the movement of the multi-needle electrode.
Preferably, the insulators of the upper electrode are placed on the sliders which allow the upper electrode to move up/down.
A circulator of the gas, which modifies the atmosphere, is placed in the reactor's chamber.
The bottom electrode is coated with dielectric material (e.g. glass, Al₂O₃) so that the reactor, ipso facto, can be used to initiate both the corona discharge and dielectric barrier discharge.

The advantage of this solution is the fact that the invention allows to carry out the sterilization process of organic products in a very flexible way. First of all, the space between the electrodes is adjustable with respect to the sterilized product (e.g. different kinds of spices), and appropriate parameters of power, voltage, and time can be selected. Furthermore, this solution allows to optimize the process by taking into account the thickness of the sterilized product, and hence its structure and density.

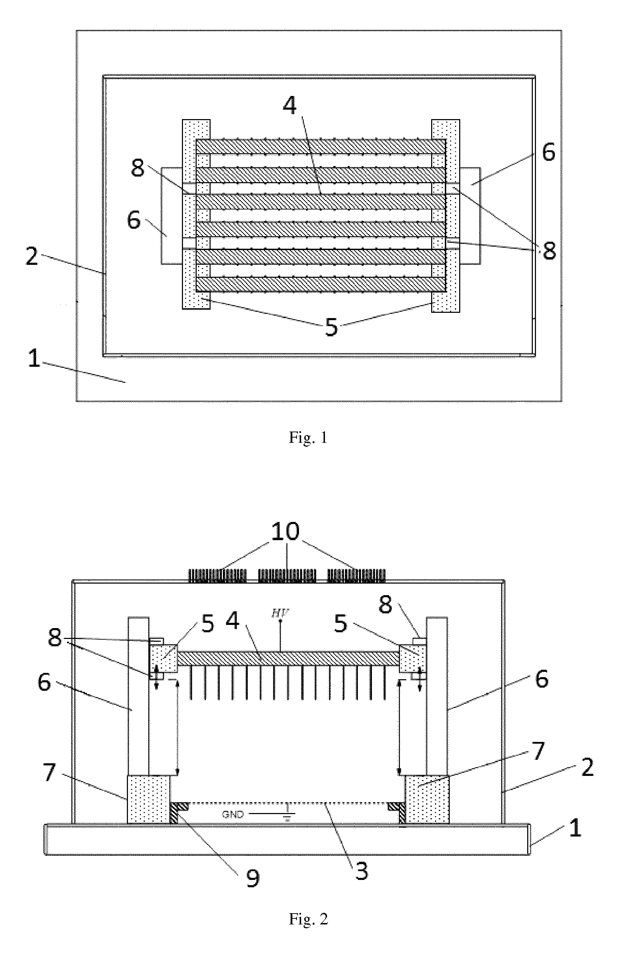

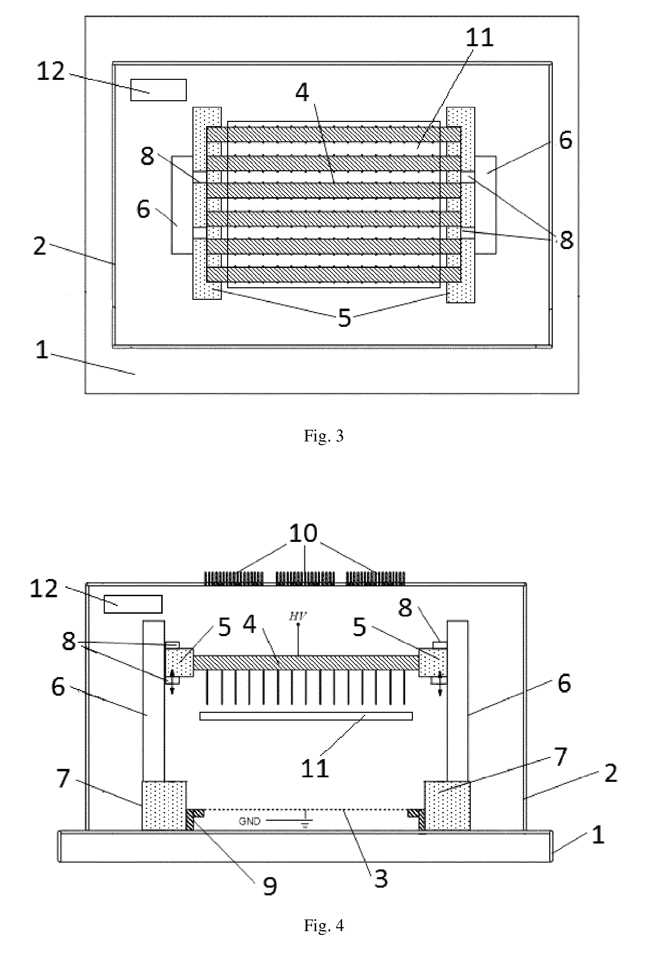

The invention is further illustrated in Figures 1-4 showing the plasma reactor chamber, top view (Fig. 1), the plasma reactor chamber, front view (Fig. 2), the plasma reactor chamber with the dielectric under the upper electrode, top view (Fig. 3), and the reactor chamber with the dielectric under the upper electrode, front view (Fig. 4).

### Example I

The non-thermal plasma reactor with power of 100 W and voltage of 0 to 30 kV, is equipped with a chamber, a base 1 on which a cover 2 of the reactor is placed. The discharge chamber is equipped with the sensors of: temperature, pressure, and humidity. The reactor has a grounding bottom electrode 3, and the upper electrode 4 (crowning HV) which is mobile and multi-needle. The upper electrode 4 is connected via the insulators 5 with the slide mechanism 6, which is placed on the base 1 on the bottom insulators 7. The bottom electrode 3 has perforations. Insulators 5 are placed in the holders-sliders 8, which are the sliders of the jack - the sliding mechanism 6. The change of the position of the electrode 4 is performed by using the drive motor in the range of 0 to 110 mm. The grounding electrode 3 is mounted in the conductivity frame 9.

Ground black pepper is placed directly in the plasma reactor without drying. Atmospheric pressure is applied and nitrogen is introduced. Next, the generator of the reactor is activated (device power 100 W, frequency 5,5 kHz) and the plasma sterilization process starts. The upper electrode 4 is lowered so that the distance between the electrodes 3 and 4 is 50 mm. The whole process lasts 11 minutes. The temperature of the process is 30°C.
The amount of the bacteria after the plasma sterilization process of the ground black pepper is below the detection limit.

### Example II (comparative)

This example is performed as described in Example I, but the upper electrode 4 is lowered so that the distance between the electrodes 3 and 4 is 80 mm. The whole process lasts 11 minutes. The temperature of the process is 30°C, device power 100 W, frequency 5,5 kHz.
The amount of the bacteria after plasma sterilization process of the ground black pepper decreases slightly, by about 1 logarithm.

### Example III

The reactor is made as described in Example I, but on the cover 2, above the upper electrode 4, there are four Peltier modules 10, and under the upper electrode 4, there is a dielectric 11. The change of the position of the electrode 4 is carried out by using the jack of the lever mechanism, made in the form of a pulley with a single stretch to ensure the uniformity of the position of the platform HV electrode.

Sweet pepper is placed directly in the plasma reactor without drying. The pressure in the reactor is not lowered. The argon is introduced. Next, the generator of the reactor is activated (device power 11 W, frequency 4,5 kHz), and the plasma sterilization process starts. The distance between the electrodes 3 and 4 is 4 mm. The whole process lasts 5 minutes. The process temperature is up to 30°C.

The amount of the bacteria after the plasma sterilization of the sweet pepper fluctuates below the detection limit.

### Example IV (comparative)

The reactor is made as described in Example III, but on the cover 2, above the upper electrode 4, there are four Peltier modules 10, and under the upper electrode 4, there is a dielectric 11. The change of the position of the electrode 4 is carried out by using the jack of the lever mechanism, made in the form of a pulley with a single stretch to ensure the uniformity of the position of the platform HV electrode.

Sweet pepper is placed directly in the plasma reactor without drying. The pressure in the reactor is not lowered. The argon is introduced. Next, the generator of the reactor is activated (device power 50 W, frequency 4,5 kHz), and the plasma sterilization process starts. The distance between the electrodes 3 and 4 is 4 mm. The whole process lasts 5 minutes. The process temperature is up to 30°C.

The amount of the bacteria after the plasma sterilization of the sweet pepper fluctuates below the detection limit, however, the sweet pepper is burned, it changes its color to black, and burning odor is noticed.

### Example V

The reactor is made as described in Example III, but on the cover 2, above the upper electrode 4, there are two fans (not illustrated in the Figures) for cooling Peltier modules 10. A circulator 12 of the gas, which modifies the atmosphere, is placed in the reactor's chamber. The bottom electrode 3 is coated with a dielectric. The change of the position of the electrode 4 is carried out by using a linear bearing with rails. Ground black pepper, is placed directly in the plasma reactor without drying. The pressure in the reactor is not lowered. Air is introduced. Next, the generator of the reactor is activated (device power 200 W, frequency 4,5 kHz) and the plasma sterilization process starts. The distance between the electrodes 3 and 4 is 100 mm. The whole process lasts 32 minutes. The process temperature is up to 30°C.
The amount of the bacteria after the plasma sterilization process of the ground black pepper is below the detection limit.

## Claims

1. The non-thermal plasma reactor for organic product sterilization, especially for spices, equipped with a chamber, a base, on which a cover of the reactor is placed, a grounding bottom electrode, and an upper electrode, characterizes in that it has a mobile upper multi-needle electrode (4) connected via insulators (5) with a slide mechanism (6), which is placed on the base (1) on the bottom insulators (7), and the lower electrode (3) has perforations, the reactors power is from 0 to 350 W, and the voltage of 0 up to 30 kV.

2. The reactor in accordance with claim 1 characterizes in that on the cover (2), above the upper electrode (4) it has at least one Peltier module (10).

3. The reactor in accordance with claim 1 characterizes in that on the cover (2), above the upper electrode (4) it has at least one fan for cooling the Peltier module (10).

4. The reactor in accordance with claim 1 characterizes in that under the upper electrode (4) there is a dielectric (11).

5. The reactor in accordance with claim 1 characterizes in that the insulators (5) of the upper electrode (4) are placed on the sliders (8).

6. The reactor in accordance with claim 1 characterizes in that it has a circulator (12) located in the chamber with gas which modifies the atmosphere.

7. The reactor in accordance with claim 1 characterizes in that the bottom electrode (3) is coated with dielectric material.
